Europäisches Patentamt

European Patent Office

Office européen des brevets

(19)

(11) Publication number: **0 230 247**
**A2**

(12) # EUROPEAN PATENT APPLICATION

(21) Application number: **87100297.8**

(22) Date of filing: **13.01.87**

(51) Int. Cl.⁴: **B01J 20/26 , A61M 1/36 ,**
**//A61K35/14**

(30) Priority: **14.01.86 JP 5535/86**
**12.03.86 JP 53959/86**

(43) Date of publication of application:
**29.07.87 Bulletin 87/31**

(84) Designated Contracting States:
**BE DE FR GB IT**

(71) Applicant: **KANEGAFUCHI KAGAKU KOGYO**
**KABUSHIKI KAISHA**
**2-4 Nakanoshima 3-chome**
**Kita-ku Osaka-shi Osaka-fu(JP)**

(72) Inventor: **Tani, Nobutaka**
**4-17-29, Fuminosato, Abeno-ku**
**Osaka-shi Osaka-fu(JP)**
Inventor: **Hayashi, Tsuneo**
**6-51, Hinoike-cho**
**Nishinomiya-shi Hyogo-ken(JP)**
Inventor: **Furuyoshi, Shigeo**
**6-6-5-31, Maikodai, Tarumi-ku**
**Kobe-shi Hyogo-ken(JP)**
Inventor: **Nagano, Youko**
**3-10, Kagoikedoori 7-chome, Chuo-ku**
**Kobe-shi Hyogo-ken(JP)**

(74) Representative: **Türk, Gille, Hrabal**
**Bruckner Strasse 20**
**D-4000 Düsseldorf 13(DE)**

(54) **Adsorbent for removing complement component.**

(57) An adsorbent for removing a complement component and/or an activated complement component, which comprises a water-insoluble porous gel having an anionic functional group at least on a part of the surface of said gel. The adsorbent can remove the complement component and the activated complement component efficiently and selectively from body fluid. Also, the activation of the complement system caused by contacting with the absorbent is not observed. Accordingly, the adsorbent of the invention is useful for removing the complement component and/or the activated complement component from body fluid in extracorporeal circulation treatment.

# ADSORBENT FOR REMOVING COMPLEMENT COMPONENT

The present invention relates to an adsorbent for removing complement components and/or activated complement components from body fluid, a removing apparatus using the same and a removing process using the same; and more particularly to an adsorbent for removing or recovering complement compononts, especially $C_{1q}$, and/or activated complement components such as $C_{3a}$, $C_{4a}$ and $C_{5a}$ from body fluid, a removing apparatus using the same and a removing process using the same.

The complement system is an important factor in humoral immune system, which is activated by an anti body reacted with an antigen and the like, and attacks and breaks antigens. However, in chronic inflammatory disease, infectious disease, autoimmune disease, neoplasm, and the like, there are often observed abnormal and undesirable immunological reactions (which are beyond normal immunological reactions and are undesirable for a living body), such as the continuous complement activation attacking patient's own cells. In the abnormal immunological reactions, there is a problem that not only it is difficult to treat but also anaphylactic shock is occasionally caused, often resulting in death of a living body. Further, in an extracorporeal circulation such as artificial dialysis, oxygenator or plasma exchange, anaphylaxis is caused due to the activation of the complement component.

In order to inhibit the abnormal complement reactions, it is required to regulate the complement system, but there is no medicine having the pharmacological action regulating the complement system in practical use. Accordingly, immunosuppressors such as steroid, which can inhibit the action of lymphocyte or arachidonic acid cascade, are used for regulating the complement system, but the immunosuppressors have various defects that there are many case reports having no effect, and the side effect cannot be avoided.

On the other hand, as a method for regulating the complement system other than the method using the medicines, there is proposed a method in which complement components and/or activated complement components are directly removed from body fluid. For instance, a method by plasma exchange is applicable to removing the complement component, and produces the desired results in some degree. However, the above method has the defects that the selectivity is low, that is, useful components in addition to the complement components are removed, and contrary results are often produced since the complement system are often activated by extracorporeal circulation.

It is an object of the present invention to provide an adsorbent for selectively and efficiently removing the complement components and/or activated complement components from body fluid without causing unnecessary activation.

A further object of the present invention is to provide an apparatus removing the complement components and/or the activated complement components using the adsorbent.

A still further object of the present invention is to provide an removing process using the adsorbent.

These and other objects of the present invention will become apparent from the description hereinafter.

In accordance with the present invention, there is provided an adsorbent for removing a complement component and an activated complement component which comprises a water-insoluble porous gel having an anionic fuctional group at least on a part of the surface of said gel.

Also, the present invention provides an apparatus for removing a complement component and/or an activated complement component using the adsorbent and a process for removing the complement component and/or the activated complement component using the adsorbent.

The complement component used in the present invention includes $C_1$, $C_2$, $C_3$, $C_4$, $C_5$, $C_6$, $C_7$, $C_8$, $C_9$, ingredients of $C_1$ such as $C_{1q}$, $C_{1r}$ and $C_{1s}$, Properdin, Factor B, Factor D, and the like. The term "activated complement component" means a component produced from the complement component by antigen-antibody reaction or other stimuluses, and the activated complement component includes an activated fragment such as $C_{2b}$, $C_{3a}$, $C_{3b}$, $C_{4a}$, $C_{5a}$ or $C_{5b}$ and an activated complex such as $C\overline{1}$, $C\overline{4b2a}$, $C\overline{4b2a3b}$ or $C\overline{3bB}$. The term "body fluid" in the instant specification means a humoral component existing in a living body, for instance, blood, plasma, serum, lymph liquid, ascites, synovia in articular cavity, fractions obtained therefrom, and the like.

In the present invention as the water-insoluble porous gel, there can be used both an organic gel and an inorganic gel, and it is suitable to use a gel scarcely adsorbing components of blood other than the desired complement component and/or the activated complement component (so-called nonspecific adsorption).

2

The representative examples of the water-insoluble porous gel are, for instance, a soft gel such as agarose, dextran or polyacrylamide; an inorganic porous material such as porous glass or porous silica gel; a synthetic polymer such as polymethyl methacrylate, polyvinyl alcohol or styrene-divinyl benzene copolymer; a porous polymer hard gel prepared from a natural polymer such as cellulose as a starting material; and the like, but the water-insoluble porous gel is not limited thereto.

The preferable water-insoluble porous gel used in the present invention is a gel having continuous pores having suitable pore size. All of the complement component and/or the activated complement component to be adsorbed are large molecules composed of polypeptide, and $C_{1q}$ is a giant molecule having a molecular weight of at least 400,000. Therefore, in order to adsorb the complement component and/or the activated complement component, it is necessary that they can easily enter into the gel.

For measuring the pore size, there are various kinds of methods, among which mercury porosimetry is most frequently employed. In case of a hydrophilic gel, however, it is difficult to apply the mercury porosimetry. In such a case, an exclusion limit is usually adopted as a measure of the pore size. The term "exclusion limit" in the present invention means, as described in the literature such as "Zikken Kosoku Ekitai Chromatography (Experimental High Speed Liquid Chromatography)", Hiroyuki Hatano and Toshihiko Hanai, published by Kabushiki Kaisha Kagaku Dojin, the minimum molecular weight of the molecule which cannot permeate into a pore, i.e. which is excluded, in a gel permeation chromatography. It is known that a value of an exclusion limit varies depending on a kind of the substances to be excluded. An exclusion limit value for globular proteins, dextran or polyethylene glycol has been quite studied. Thus, in the present invention, a value of an exclusion limit measured by using globular proteins and/or viruses, which are regarded as the most similar substances to the complement component and the activated complement component, is suitably employed.

As the result of the investigation using a variety of water-insoluble porous gels having a different value of an exclusion limit, it is unexpectedly shown that a gel having an exclusion limit value of about $2 \times 10^5$, which is smaller than the molecular weight of the complement component, can adsorb the complement component in some extent and that a gel having a larger pore size does not always exhibit an increased capacity of adsorbing but, conversely, it is observed that an adsorbing capacity of such gel decreases or proteins other than the complement component are likely to be adsorbed, which means that there exist a suitable range of a pore size.

That is, it is found that a water-insoluble porous gel having an exclusion limit of less than $2 \times 10^5$ can hardly adsorb the complement component and is not suited for practical use, whereas a water-insoluble porous gel having an exclusion limit of $2 \times 10^5$ to $5 \times 10^5$, which is nearly the molecular weight of the complement component, can adsorb the complement component to some extent. Subsequenty, it is observed that an amount of an adsorbed complement component increases with the increase of an exclusion limit, by and by reaching its top, and it extremely decreases when an exclusion limit is over $5 \times 10^7$ because the surface area of the gel is too small. Therefore, an exclusion limit of a water-insoluble porous gel used for adsorbing the complement component is from $2 \times 10^5$ to $5 \times 10^7$, preferably from $1 \times 10^6$ to $3 \times 10^7$.

When the activated complement component is adsorbed, the same phenomenon as mentioned above occurs. Therefore, in such a case, and exclusion limit of a water-insoluble porous gel is from $1 \times 10^4$ to $1 \times 10^6$, preferably from $3 \times 10^4$ to $7 \times 10^5$.

With respect to a porous structure of the water-insoluble porous gel, a structure uniformly having pores at any part of the gel is more preferable than a structure having pores only on the surface of the gel. It is preferred that a porosity of the gel is not less than 20 %. The shape of the gel may be selected from any shapes such as particle, fiber, sheet and hollow fiber. When using the water-insoluble porous gel in the shape of particle, a particle having a particle size of 1 to 5000 μm is preferred.

In the invention, any anionic functional groups can be used so long as the groups are charged with negative electricity in pH value around neutrality. Representative examples of the anionic functional group are, for instance, carboxyl group, sulfonic acid group, sulfate group, silanol group, phosphate group, phenolic hydroxyl group, and the like, but the groups are not limited thereto. Polyanions having a plurality of the above anionic functional groups can be used as well as monoanionic compounds.

The compound containing sulfate group and the polyanions having the above functioned groups are the most preferable, since they have a strong affinity for the complement component and/or the activated complement component.

Representative examples of the polyanion are, for instance, a synthetic polyanion such as polyacrylic acid, polyvinyl sulfonic acid, polyvinyl phosphoric acid, polystyrenesulfonic acid, polystyrenephosphoric acid, polyglutamic acid, polyaspartic acid, polymethacrylic acid, polyphosphoric acid or styrene-maleic acid copolymer; an anionic functional group-containing polysaccharide such as heparin, dextran sulfate, chondroitin, chondritin sulfate, chitin or chitosan; and the like, but the polyanions are not limited thereto.

There are various methods for introducing the anionic functional groups into the adsorbent and any methods can be applied to the invention. As methods for introducing the anionic functional group into the adsorbent, there are exemplified, for instance,

(I) a method in which monomers or crosslinking agents having the anionic functional group or a group capable of easily converting into the anionic functional group are polymerized to form the adsorbent,

(2) a method in which the anionic functional group-containing compound is immobilized on a water-insoluble carrier,

(3) a method in which a compound capable of forming the anionic functional group is directly reacted with a water-insoluble carrier, and the like. Of course, the anionic functional group-containing compound having the anionic functional group in itself such as glass, silica and alumina may be used as the adsorbent

In the method (I), examples of the monomers or crosslinking agents are, for instance, acrylic acid, acrylic acid ester, methacrylic acid, methacrylic acid ester, styrenesulfonic acid, and the like, but the monomers or crosslinking agents are not limited thereto.

In the method (2), various immobilizing methods can be applied, but an immobilizing method capable of forming a strong covalent bond is preferable.

As compounds having sulfate group among the anionic functional group-containing compound used for introducing the anionic functional group by immobilization, there are exemplified, for instance, sulfuric acid esters of hydroxyl group-containing compounds such as alcohol, saccharose and glycol. Among them, there is preferred sulfuric acid esters having a functional group which can be utilized for the immobilization onto the water-insoluble porous gel in addition to sulfate group. Among the above sulfuric acid esters, partially sulfated polyhydric alcohols, and a particularly sulfated saccharide are preferable, since the sulfuric acid esters not only have both sulfate group and the functional group necessary for the immobilization but also are high in the biocompatibility and the activity. A sulfated polysaccharide is more preferable since it can be easily imobilized onto the water-insoluble porous gel. As to the anionic functional group-containing compound other than the compound having sulfate group, a compound having a functional group which can be utilized in immobilization in addition to the anionic functional group is preferable.

As the representative example of the method (3), there are, for instance, a method in which sulfate group is introduced into a water-insoluble carrier having hydroxyl group, and the like. In such a case, sulfate group can be directly introduced into the adsorbent by reacting a reagent such as chlorosulfonic acid or concentrated sulfuric acid with the water-insoluble carrier having hydroxyl group.

It is preferable that the amount of the introduced anionic functional group is from 0.0I $\mu$mol to I0 mmol per I ml of the adsorbent. When the amount is less than 0.0I $\mu$mol, a sufficient adsorbing capacity cannot be obtained. When the amount is more than I0 mmol, nonspecific adsorption increases, which make the adsorbent unsuitable for a practical usage.

There are various ways as the method for removing the complement component and/or the activated complement component from body fluid by using the adsorbent according to the present invention, and the adsorbent can be used in any of methods. There is preferable a method in which body fluid is passed through an apparatus comprising a container having an inlet and an outlet for body fluid and the adsorbent of the invention packed in the container, since the method is easy.

The present invention is more specifically described and explained by means of the following Examples and Comparative Examples in which all % and parts are by weight unless otherwise noted. It is to be understood that the present invention is not limited to the Examples and Comparative Examples, and various changes and modifications may be made in the invention without departing from the spirit and scope thereof.

Example 1

There were dried 10 mℓ of CK gel A-3 (a commercially available porous cellulose gel made by Chisso Corporation, exclusion limit of spherical proteins: 5 $\times$ 10$^7$, particle size: 45 -105 μm) and 10 mℓ of Cellulofine GCL-2000 (a commercially available porous cellulose gel made by Chisso Corporation, exclusion limit of spherical proteins: 3 $\times$ 10$^6$, particle size: 45 -105 μm, crosslinked gel) by a critical point drying method in ethanol, separately. Each of the dried gels was suspended in 10 mℓ of pyridine sufficiently dehydrated and the suspension was cooled with ice, to which 2 mℓ of chlorosulfonic acid was added dropwise with stirring, the stirring being continued for 10 minutes after the dropwise addition was completed.

After completion of the reaction, the reaction mixture was filtered and washed with pyridine and then water to give a cellulose gel on whose surface sulfate ion was introduced.

Example 2

After 10 mℓ of CK gel A-4 (a commercially available porous cellulose gel made by Chisso Corporation, exclusion limit of spherical proteins: 4 $\times$ 10$^7$) was washed with water, the gel was filtered by suction, to which 6 mℓ of dimethyl sulfoxide, 2.6 mℓ of 2N NaOH and 1.5 mℓ of epichlorohydrin were added, and the mixture was stirred at 40°C for 2 hours. After the reaction was completed, the gel was filtered off from the reaction mixture and was washed with water to give a cellulose gel on which epoxy ion was introduced. To the obtained cellulose gel was added 6 mℓ of concentrated aqueous ammonia and the mixture was reacted at 40°C for 2 hours to give an aminated cellulose gel.

To 2 g of the obtained aminated cellulose gel was added a solution of 4 g of dextran sulfate sodium having a molecular weight of about 5000 in 8 mℓ of buffer solution of 0.1 M phosphoric acid (pH 8) and the mixture was shaken at room temperature for 16 hours. After completing the reaction, 20 mg of NaCNBH$_3$ was added to the reaction mixture, which was stirred at room temperature for 30 minutes and heated at 40°C for 4 hours. Then, the gel was filtered off from the reaction mixture and washed with water to give a cellulose gel with immobilized dextran sulfate.

Example 3

There were added 4 g of 20 % NaOH, 12 g of heptane and one drop of the nonionic surfactant TWEEN 20 (a commercially available polyoxyethylene sorbitol fatty acid ester made by Kao Atlas Kabushiki Kaisha) to 10 mℓ of CK gel A-3. The mixture was stirred at 40°C for 2 hours, to which 5 g of epichlorohydrin was added and the resultant was stirred at 40°C for 2 hour. The gel was filtered off from the reaction mixture and washed with water to give an epoxidated cellulose gel.

To 5 mℓ of the obtained gel were added 3 g of dextran sulfate sodium and 5 mℓ of water, the pH of the mixture was adjusted to pH 9 and the mixture was shaken at 45°C for 16 hours. Then, the gel was filtered off from the reaction mixture and was washed with water, the unreacted epoxy group in the gel was blocked by adding 0.5 % aqueous solution of monoethanolamine to the gel and shaking to give a cellulose gel with immobilized dextran sulfate sodium.

Example 4

An aminated cellulose gel was prepared in the same manner as in Example 2.

To 5 mℓ of the obtained aminated gel was added a solution formed by dissolving 0.2 g of sodium polyacrylate having a molecular weight of 190,000 to 500,000 into 10 mℓ of water and adjusting the pH value to 4.5. To the mixture was added 200 mg of 1-ethyl-3-(dimethylaminopropyl)carbodiimide while maintaining the pH value of 4.5, which was shaked at 4°C for 24 hours. After completion of the reaction, the gel was filtered off from the reaction mixture and washed with water to give a cellulose gel with immobilized polyacrylic acid.

5

## Example 5

The procedure of Example I was repeated except that Cellulofine GC 700 (a commercially available porous cellulose gel made by Chisso Corporation, exclusion limit of spherical proteins: $4 \times 10^5$, particle size: 45 -105 μm) was employed as the cellulose gel to give a cellulose gel on whose surface sulfate ion was introduced.

[Concentration of the complement component and the activated complement component]

A test tube was charged with I mℓ of each of the adsorbents obtained in Examples I to 5, to which 4 mℓ of normal human serum was added, and the mixture was shaken at 37°C for 2 hours.

Concentrations of the complement components and the activated complement components in supernatant liquid were measured. The concentrations of $C_{1q}$, $C_2$ and $C_6$ were measured according to SRID method (single radial immunodiffusion method), and the concentrations of $C_{3a}$ and $C_{5a}$ were measured according to RID (radioimmunoassay).

The results are shown in Table I.

## Comparative Example I

A test tube was charged with 4 mℓ of normal human serum and it was shaken at 37°C fo 2 hours.

Concentrations of the complement component and the activated complement component in supernatant liquid were measured in the same manner as above.

The results are shown in Table I.

## Table 1

| | Adsorbent | Complement component | | | Activated complement component | |
|---|---|---|---|---|---|---|
| | | $C_{1q}$ (%) | $C_2$ (%) | $C_6$ (%) | $C_{3a}$ (ng/ml) | $C_{5a}$ (ng/ml) |
| Ex. 1 | Sulfated CKA-3 | 37 | 72 | 68 | 15 | 10 |
| | Sulfated GCL-2000 | 46 | 62 | 53 | 12 | 8 |
| Ex. 2 | Dextran sulfate-aminated CKA-4 | 52 | 80 | 90 | 10 | 7 |
| Ex. 3 | Dextran sulfate-expoxidated CKA-3 | 55 | 83 | 92 | 9 | 7 |
| Ex. 4 | Polyacrylic acid-CKA-3 | 22 | 36 | 46 | 13 | 12 |
| Ex. 5 | Sulfated GC700 | 85 | 65 | 74 | 11 | 10 |
| Com.Ex.1 | No | 95 | 96 | 102 | 21 | 9 |

0 230 247

As shown in Table I, it is observed that the complement components, especially $C_{1q}$, are efficiently removed by using the adsorbent of the invention. Also, the activation of the complement system caused by contacting the complement component with the adsorbent of the invention is not observed (and the activation is rather inhibited).

Example 6

To l0 ml of Toyopearl HW 75 (a commercially available crosslinked polyacrylate gel made by Toyo Soda Manufacturing Co., Ltd., exclusion limit of proteins: $5 \times 10^5$, particle size: 50 -l00 μm), which was a hard gel having pores at any part thereof, were added 6 ml of saturated aqueous solution of NaOH and l5 ml of epichlorohydrin, and the mixture was reacted at 40°C for 2 hours with stirring to give an epoxidated gel. To the obtained gel was added 20 ml of concentrated aqueous ammonia and the mixture was stirred at 50°C for 2 hours to introduce amino group into the gel.

There was added 5 ml of the gel into which amino group was introduced, to l5 ml of l0 % aqueous solution of polyacrylic acid and the pH of the mixture was adjusted to pH 4.5, to which 300 mg of l-ethyl-3-(dimethylaminopropyl)carbodiimide was added while maintaining the pH value of 4.5 and the mixture was shaken at 4°C for 24 hours. After completing the reaction, the gel was washed with in order of 2 M saline aqueous solution, 0.5 M saline aqueous solution and water to give a gel with immobilized polyacrylic acid.

Example 7

There was added l0 ml of CK gel A-3 to l0 ml of water, to which 5 ml of 2 M NaCl and l.8 ml of epichlorohydrin were added, and the mixture was reacted at 40°C for 2 hours with stirring. After completing the reaction, the gel was filtered and washed with water to give an epoxidated cellulose gel.

To 5 ml of the obtained gel was added 3 g of dextran sulfate sodium and 5 ml of water, the pH of the mixture being adjusted to pH 9 and the mixture was shaken at 45°C for l6 hours. Then, the gel was filtered off from the reaction mixture, and the gel was washed with in order of 2 M saline aqueous solution, 0.5 M saline aqueous solution and water to give a cellulose gel with immobilized dextran sulfate sodium.

Example 8

There was dried l0 ml of Cellulofine GCL 2000 by a critical point drying method in ethanol. The dried gel was suspended in l0 ml of pyridine sufficiently dehydrated and the suspension was cooled with ice, to which 2 ml of chlorosulfonic acid was added dropwise with stirring, the stirring being continued for l0 minutes after the dropwise addition was completed. After completing the reaction, the gel was filtered off from the reaction mixture and washed with pyridine and then water to give a cellulose gel on whose surface sulfuric acid residue was introduced.

With respect to each of the adsorbends prepared in Examples 6 to 8, the adsorption capacity to the activated complement component was estimated as follows:

Blood was gathered from a normal human and serum was separated from the gathered blood by centrifuging. A vial made from polystyrene was charged with the obtained serum, the serum was shaken and was passed several times through a membrane filter made from cellulose acetate to activate the complement system.

After washing each of the adsorbent obtained in Examples 6 to 8 with physiological saline to equilibrate, a glass test tube was charged with l ml of the adsorbent, to which 5 ml of the serum having the activated complement component was added, and the mixture was incubated at 37°C for l hour. After incubating, the adsorbent was separated by centrifuging and concentrations of $C_{3a}$ and $C_{5a}$ in supernatant were measured according to RIA.

The results are shown in Table 2.

Comparative Example 2

There was incubated 5 mℓ of the serum having the activated complement component obtained in the same manner as mentioned above at 37°C for I hour without adding the adsorbent. Then, concentrations of $C_{3a}$ and $C_{5a}$ in supernatant were measured by using in the same manner as mentioned above.

The results are shown in Table 2.

Table 2

|  | Concentration of $C_{3a}$ (ng/mℓ) | Concentration of $C_{5a}$ (ng/mℓ) |
|---|---|---|
| Ex. 1 | 702 | 80 |
| Ex. 2 | 32 | 17 |
| Ex. 3 | 43 | 33 |
| Com.Ex.2 | 2050 | 145 |

Example 9

A polypropylene minicolumn was filled with 5 mℓ of the adsorbent obtained in Example 7, and the adsorbent was thoroughly washed with physiological saline. Human plasma was obtained by plasma separation using membrane plasma separator (made by Asahi Medical Kabushiki Kaisha) during a plasma exchange treatment. There was passed 30 mℓ of the otained plasma through the column at a rate of I mℓ/minute and concentrations of $C_{3a}$ and $C_{5a}$ in the plasma obtained before and after the column treatment were, respectively, measured in the same manner as in Examples 6 to 8.

Table 3

|  | Concentration of $C_{3a}$ in the plasma (ng/mℓ) | Concentration of $C_{5a}$ in the plasma (ng/mℓ) |
|---|---|---|
| Before the column treatment | 2230 | 82 |
| After the column treatment | 41 | 30 |

As shown in Examples, when the adsorbent of the invention is used, the complement component, especially $C_{1q}$, and the activated complement component are efficiently removed. On the other hand, the activity of the complement component system caused by contacting with the adsorbent of the invention is not observed. Accordingly, the adsorbent of the invention is useful as the adsorbent for removing the complement component and/or the activated complement component from body fluid in extracorporeal circulation treatment.

## Claims

1. An adsorbent for removing a complement component and/or an activated complement component, which comprises a water-insoluble porous gel having an anionic functional group at least on a part of the surface of said gel.

2. The adsorbent of Claim 1, wherein said gel comprises a hydroxyl group-containing compound.

3. The adsorbent of Claim 1, wherein said anionic functional group is at least one group selected from the group consisting of carboxyl group, sulfonic acid group and sulfate group.

4. The adsorbent of Claim 1, wherein an anionic functional group-containing compound is immobilized on said gel with covalent bond.

5. The adsorbent of Claim 1, which has an exclusion limit suitable for removing the complement component within the range of $2 \times 10^5$ to $5 \times 10^7$.

6. The adsorbent of Claim 1, which has an exclusion limit suitable for removing the activated complement component within the range of $1 \times 10^4$ to $1 \times 10^6$.

7. The adsorbent of Claim 4, wherein said anionic functional group-containing compound contains at least one anionic functional group selected from the group consisting of carboxyl group, sulfonic acid group and sulfate group.

8. The adsorbent of Claim 4, wherein said anionic functional group-containing compound is a polyanion.

9. The adsorbent of Claim 1, wherein a sulfate group is introduced into said gel by sulfuric esterification of hydroxyl group in a hydroxyl group-containing water-insoluble porous gel.

10. The adsorbent of Claim 1, wherein said gel comprises a sulfate group-containing compound.

11. The adsorbent of Claim 10, wherein said sulfate group-containing compound is sulfated polysaccharide.

12. An apparatus for removing a complement component and/or an activated complement component from body fluid, which comprises a container having a inlet and an outlet for body fluid and an adsorbent packed in said container, said adsorbent comprising a water-insoluble porous gel having an anionic functional group at least on a part of the surface of said gel.

13. A process for removing a complement component and/or an activated complement component from body fluid which comprises

passing body fluid containing a complement component and/or an activated complement component through a removing apparatus,

said apparatus comprising a container having a inlet and an outlet for body fluid and an adsorbent packed in said container comprising a water-insoluble porous gel having an anionic functional group at least on a part of the surface of said gel.

14. The process of Claim 13, wherein a sulfate group is introduced into said gel by sulfuric esterification of hydroxyl group in a hydroxyl group-containing water-insoluble porous gel.